# EUROPÄISCHE PATENTSCHRIFT

(11) **EP 3 630 471 B1**
(45) Veröffentlichungstag und Bekanntmachung des Hinweises auf die Patenterteilung: **21.08.2024**
(21) Anmeldenummer: 18725508.8
(22) Anmeldetag: 17.05.2018
(51) Int. Cl.: B29D 11/00, A61F 2/16, G02C 7/02, B29D 11/02

(54) **KÜNSTLICHE AUGENLINSE MIT LASERERZEUGTER DOPPELBRECHENDER STRUKTUR SOWIE VERFAHREN ZUM HERSTELLEN EINER KÜNSTLICHEN AUGENLINSE**
ARTIFICIAL EYE LENS WITH LASER-GENERATED BIREFRINGENT STRUCTURE AND METHOD FOR PRODUCING AN ARTIFICIAL EYE LENS
CRISTALLIN ARTIFICIEL À STRUCTURE BIRÉFRINGENTE PRODUITE PAR LASER ET PROCÉDÉ DE FABRICATION D'UN CRISTALLIN ARTIFICIEL

(30) Priorität: 01.06.2017 DE 102017112087
(43) Veröffentlichungstag der Anmeldung: 08.04.2020
(73) Patentinhaber: Carl Zeiss Meditec AG, 07745 Jena (DE)
(72) Erfinder: KASCHKE, Michael, 73447 Oberkochen (DE); DICK, Manfred, 07926 Gefell (DE); GERLACH, Mario, 16548 Glienicke-Nordbahn (DE)
(74) Vertreter: Hofstetter, Schurack & Partner
(86) Internationale Anmeldenummer: PCT/EP2018/062924
(87) Internationale Veröffentlichungsnummer: WO 2018/219671

(56) Entgegenhaltungen:
- EP-A2- 1 250 609
- WO-A1-94/27169
- WO-A2-2012/138426
- US-A- 5 100 226
- US-A- 5 142 411
- US-A1- 2009 287 306
- US-A1- 2015 378 065
- PIN YANG ET AL: "Femtosecond laser-pulse-induced birefringence in optically isotropic glass", JOURNAL OF APPLIED PHYSICS, vol. 95, no. 10, 15 May 2004 (2004-05-15), US, pages 5280 - 5283, XP055495603, ISSN: 0021-8979, DOI: 10.1063/1.1707231

## Beschreibung

### Technisches Gebiet

Die Erfindung betrifft eine künstliche Augenlinse mit einem optischen Teil, der eine in Richtung einer optischen Hauptachse der künstlichen Augenlinse betrachtet erste optische Seite und eine gegenüberliegende zweite optische Seite aufweist, wobei der optische Teil eine doppelbrechende Struktur aufweist, die zur optischen Abbildungseigenschaft des optischen Teils beiträgt. Des Weiteren betrifft die Erfindung auch ein Verfahren zum Herstellen einer derartigen künstlichen Augenlinse mittels eines Lasers.

### Stand der Technik

Multifokale, künstliche Augenlinsen sind aus dem Stand der Technik vielfältigst bekannt. Insbesondere sind dazu Intraokularlinsen bekannt, die die natürliche Linse im Auge ersetzen und diesbezüglich implantiert sind.

Aus der WO 94/27169 A1 ist eine Intraokularlinse bekannt, die ein doppelbrechendes Material in Form eines Flüssigeinkristall-Elastomers oder eines Flüssigeinkristall-Duromers aufweist.

Aus der US 2010/0082017 A1 ist eine Intraokularlinse bekannt, bei welcher in einem haptischen Teil als auch in einem optischen Teil Schlitze ausgebildet sind, um die mechanische Charakteristik und auch die Strukturcharakteristik der Linse zu modifizieren. Diese länglichen Schlitze sind im Inneren der Intraokularlinse ausgebildet, insbesondere mit einem Laser.

Darüber hinaus ist aus der US 2004/0032566 A1 ein Verfahren zur Markierung einer Intraokularlinse mittels Laser bekannt. Mit dem Laser wird eine Mikroperforierung des optischen Teils der Linse durchgeführt.

Darüber hinaus ist aus der US 2014/0135920 A1 ein Herstellungsverfahren für eine Intraokularlinse bekannt, bei welchem mit einem Laserstrahl eines Ultrakurzpulslasers das hydrophile Verhalten des Polymerwerkstoffes, aus dem die künstliche Augenlinse bereits hergestellt ist, verändert wird. Durch diese Veränderung des hydrophilen Verhaltens des Polymerwerkstoffes wird eine Reduktion des optischen Brechungsindexes dieses Polymermaterials erzeugt.

Als weitere bekannte Herstellungsmöglichkeit ist beispielsweise eine Ultrapräzisionsbearbeitung genannt. Dort wird mit einem monokristallinen Diamantwerkzeug gearbeitet, indem dieses Werkzeug mechanisch auch direkt auf den Kunststoff, aus welchem die Augenlinse erzeugt werden soll, einwirkt. Diese Technologie arbeitet mit geometrisch bestimmtem, monokristallinem Diamantwerkzeug entsprechend den klassischen spanenden Verfahren, wie Drehen oder Fräsen. Für diese Herstellungsverfahren sind jedoch sehr stabile Maschinen und gleichbleibende Umgebungsbedingungen erforderlich. Typischerweise ist hier die Fertigungsumgebung klimatisiert und schwingungsgedämpft. Daher ist die Herstellung sehr aufwendig.

Mithilfe der Ultrapräzisionsbearbeitung können neben der oben genannten direkten Bearbeitung der Kunststoffoptiken auch Abformwerkzeuge bereitgestellt werden, mit denen dann in einem Gieß-Prozess die Augenlinse abgeformt werden kann, was auch kostengünstiger ist.

Die Herstellung von sehr komplexen Profilen an der Oberfläche eines optischen Teils ist dabei jedoch eingeschränkt und das Herstellen von optisch wirksamen Strukturen im Inneren des optischen Teils nicht möglich. Mit einem Laser erzeugte Strukturen haben demgegenüber Vorteile.

Bei bekannten künstlichen Augenlinsen, insbesondere bei Intraokularlinsen, treten unerwünschte Beugungsordnungen auf, die deren optische Funktionalität einschränken. Diese unerwünschten Beugungsordnungen treten auch unabhängig von den bekannten Herstellungsverfahren mehr oder minder stark auf. Gerade Halos und Glares sind hier als störende Beeinträchtigungen zu nennen. Als Halos werden Lichteffekte bezeichnet, die durch Brechung und Reflexion von Licht beispielsweise Lichthöfe um Lampen, Scheinwerfer und andere Lichtquellen erzeugen. Sie treten auch als Lichtringe auf. Gerade bei Dämmerung oder in der Nacht sind dies besonders unerwünschte Effekte die auch zur Fehlwahrnehmung führen können. Gerade bei Dämmerung und in der Nacht treten Halos bei relativ scharfen Hell-Dunkel-Übergängen auf, wodurch neben einem Blenden eines Auges allgemein auch das Sehen für einen Augenlinsenträger relativ anstrengend ist. Als Glares werden Blendeffekte bezeichnet. Sie treten insbesondere bei direkter Einstrahlung von hellem Licht auf, beispielweise in der Dämmerung oder in Dunkelheit, wenn eine helle Lichtquelle in Richtung des Beobachters strahlt. Auch direkt eingestrahltes oder reflektiertes Sonnenlicht erzeugt derartige Blendeffekte.

Aus der DE 38 55 963 T2 ist eine doppelbrechende Intraokularlinse bekannt, die im optischen Teil aus mehreren separaten Teilen aufgebaut ist. Dies ist sehr aufwendig und die Funktionalität der Doppelbrechung ist stark eingeschränkt.

### Darstellung der Erfindung

Es ist Aufgabe der Erfindung, eine künstliche Augenlinse und ein Verfahren zum Herstellen einer künstlichen Augenlinse zu schaffen, bei welcher bzw. mit welchem eine doppelbrechende Struktur einfacher und dennoch funktioneller ausgebildet ist.

Ein Aspekt der Erfindung betrifft eine künstliche Augenlinse, die einen optischen Teil aufweist, mittels welchem die optische Abbildungseigenschaft der Augenlinse charakterisiert ist. Dieser optische Teil weist eine erste optische Seite und eine in Richtung der optischen Hauptachse dieser Augenlinse betrachtet gegenüberliegende zweite optische Seite auf. Die künstliche Augenlinse weist des Weiteren insbesondere eine Haptik auf. Mit der Haptik ist die Augenlinse positionell in einem Auge gehalten. Die künstliche Augenlinse weist zusätzlich oder anstatt der Haptik eine den optischen Teil zumindest bereichsweise umgebende und zur Haptik unterschiedliche Einfassung auf. Diese Einfassung ist dann weder Bestandteil des optischen Teils noch ist sie Bestandteil insbesondere von Bügeln einer Haptik, wenn eine derartige Haptik vorhanden ist.

Der optische Teil der künstlichen Augenlinse ist einstückig ausgebildet und somit insbesondere auch einstückig hergestellt. Die künstliche Augenlinse weist am oder im einstückigen optischen Teil selbst und somit in nur einem als einziges Teil bereitgestellten optischen Teil eine doppelbrechende Struktur auf, die darüber hinaus auch noch als spezifische Herstellungsstruktur ausgebildet ist, nämlich als Laserstruktur. Dadurch ist die Ausgestaltung eines optischen Teils einer künstlichen Augenlinse bauteilminimiert und dennoch die doppelbrechende Struktur präzisiert und somit bezüglich ihrer Abbildungseigenschaften genauer. Durch die Ausgestaltung als Laserstruktur kann sie sowohl örtlich sehr vielfältig am oder im optischen Teil ausgebildet werden und auch bezüglich ihrer Form und/oder Orientierung sehr exakt und auch filigran ausgebildet werden. Damit ist auch die optische Funktionsflexibilität für eine derartige doppelbrechende Struktur wesentlich erhöht.

Die Doppelbrechung ist die Fähigkeit von optisch anisotropen Medien, ein Lichtbündel in zwei senkrecht zueinander polarisierte Teilbündel zu trennen. Die Ursache dieses Effekts liegt im unterschiedlichen Brechungsindex in Abhängigkeit von der Ausbreitungsrichtung und Polarisation des Lichtes. Auch isotrope Materialien können durch entsprechende Bearbeitung, auch hier insbesondere durch spezifische Laserstrahlen, nämlich ultrakurze Laserpulse, ganz individuell anisotrop werden und dann spezifisch doppelbrechend werden.

Durch die beschriebenen zwei Methoden (gepulster Laserbetrieb und ultrakurz gepulster Laserbetrieb) wird auch eine besonders exakte Vorzugsrichtung in eine künstliche Augenlinse eingearbeitet, die ansonsten die künstliche Augenlinse nicht ändert, aber eine sehr genaue polarisationsabhängige Transmission ermöglicht.

Die Herstellung ist vorzugsweise auch für trifokale künstliche Augenlinsen, wie Intraokularlinsen, anwendbar, um diese polarisierungsabhängig zu machen. Foki lassen sich dann durch Polarisationsfilter ausschalten. Diese könnten dann beispielsweise in einer Brille enthalten sein. Insbesondere ist die doppelbrechende Struktur derart ausgebildet, dass eine Lichtverteilung auch auf zumindest zwei verschiedene Foki der künstlichen Augenlinse moduliert ist.

Insbesondere ist das Material des optischen Teils, in das die doppelbrechende Struktur ausgebildet ist, ein isotropes Material. Gerade durch die Ausgestaltung als Laserstruktur kann auch ein derartiges Material doppelbrechend verändert werden.

Vorzugsweise ist die doppelbrechende Struktur zumindest bereichsweise eine lineare doppelbrechende Struktur. Dadurch wird eine linear polarisierende Wirkung dieser doppelbrechenden Struktur erhalten. Dadurch können relativ einfach ganz spezifische Doppelbrechungsarten erreicht werden, die präzise optische Funktionalität aufweisen.

Insbesondere wird die doppelbrechende Struktur mit einem gepulsten Laserstrahl so erzeugt, dass lineare Strukturen in dem optischen Teil mit diesem gepulsten Laserstrahl bis kurz vor dem Schmelzpunkt aufgeheizt werden. Dabei ist insbesondere ein gepulster Laserstrahl mit einer Pulslänge zwischen 1 ns und 1 s, einer Wellenlänge zwischen 320 nm und 11 µm, einer Pulswiederholrate zwischen 0,5 Hz und 1kHz, einem Fokusdurchmesser von einigen µm vorgesehen, um durch lineare Absorption das Material kurzzeitig zu erwärmen.

Eine weitere Möglichkeit, die zusätzlich oder anstatt dazu durchgeführt werden kann, ist darin zu sehen, dass ultrakurze Laserpulse so erzeugt werden, dass mit diesen Laserpulsen nicht lineare Wechselwirkungen innerhalb der linearen Strukturen in dem optischen Teil bis kurz vor der Laserdisruptionsschwelle erzeugt werden.

Weiterhin kann vorgesehen sein, dass mittels der ultrakurzen Laserpulse Monomer-Moleküle, aus dem die künstliche Augenlinse zusammengesetzt ist, gezielt ausgerichtet werden. Diese Ausrichtung ist insbesondere senkrecht oder in einem Winkel zu einer optischen Achse der Laser-Vorrichtung vorgesehen, welche die ultrakurzen Laserpulse aussendet. So kann diese Ausrichtung durch Einstrahlung eines weiteren Laserstrahls in das Fokusgebiet der Laser-Vorrichtung zur Multiphotonen-Polymerisation erfolgen. Weiterhin kann ein elektrisches Feld und oder ein magnetisches Feld zur Ausrichtung der Monomer-Moleküle verwendet werden, bevor eine Polymerisation erfolgt.

Insbesondere ist die künstliche Augenlinse mit einer doppelbrechenden Struktur ausgebildet, so dass eine zumindest bifokale künstliche Augenlinse mit zumindest reduzierten Halos und reduzierten Glares ausgebildet ist.

Die künstliche Augenlinse weist insbesondere zusätzlich zu der doppelbrechenden Struktur vorzugsweise zumindest eine diffraktive Gitterstruktur auf, die zur optischen Abbildungseigenschaft des optischen Teils beiträgt. Die diffraktive Gitterstruktur ist im optischen Teil der künstlichen Augenlinse ausgebildet. Diese diffraktive Gitterstruktur ist insbesondere ein Amplitudengitter, welches darüber hinaus in dem insbesondere einstückig ausgebildeten optischen Teil der künstlichen Augenlinse als Laserstruktur ausgebildet ist. Ein Amplitudengitter ist ein absorbierendes Gitter. Ein Amplitudengitter ist derart ausgebildet, dass es die Amplitude der einfallenden Lichtwelle moduliert. Ein Amplitudengitter ist ein optisches Gitter, welches einfallendes Licht teilweise absorbiert.

Ein Amplitudengitter kann als Transmissionsgitter oder als Reflexionsgitter ausgebildet sein. Durch die Ausgestaltung eines derartig spezifischen optischen Gitters als Laserstruktur ist es einerseits äußerst präzise herstellbar, andererseits kann es lokal sehr definiert an unterschiedlichen Stellen des optischen Teils erzeugt werden. Durch die spezifische Ausgestaltung eines derartigen Amplitudengitters können unerwünschte Beugungsordnungen der künstlichen Augenlinse unterdrückt werden. Damit wird insbesondere auch die Kontrastverbesserung des optischen Systems ermöglicht. Insbesondere ist es durch eine derartige Ausgestaltung einer diffraktiven Gitterstruktur als Amplitudengitter, welches als Laserstruktur ausgebildet ist, auch möglich, Halos und Glares in besonders vorteilhafter Weise zumindest deutlich zu reduzieren. Unerwünschte Blendungen und Reflexionseffekte, wie sie durch diese spezifischen, optisch störenden Effekte auftreten, können dadurch deutlich reduziert werden.

In einer vorteilhaften Ausführung ist das Amplitudengitter als Mikroperforation in dem optischen Teil ausgebildet. Dies ist eine besonders vorteilhafte Ausführung, wenn ein derartig spezifisches optisches Gitter als Laserstruktur erzeugt ist. Wird durch eine Mikroperforation ein Amplitudengitter zugestaltet, ermöglicht dies eine höchst präzise Ausgestaltung der einzelnen Strukturbereiche dieses Amplitudengitters. Dadurch wird die optische Wirkung des Amplitudengitters in besonders vorteilhafter Weise erreicht. Andererseits wird durch eine derartige Ausgestaltung eine sehr konturenscharfe Ausgestaltung der Konturbereiche des Amplitudengitters erreicht, sodass gerade auch an Randbereichen des Amplitudengitters nicht mehr zum Amplitudengitter zugehörige Bereiche des optischen Teils nicht unerwünscht beeinträchtigt werden und deren optische Abbildungseigenschaft daher nicht unerwünscht verfälscht wird. Gerade durch die Ausgestaltung als Mikroperforation lassen sich auch sehr feindosierte Unterschiede in den Strukturbereichen des Amplitudengitters herstellen, sodass auch hier sehr individuelle optische Abbildungseigenschaften des Amplitudengitters in sich selbst geschaffen werden können.

Durch eine Ausgestaltung des Amplitudengitters als Mikroperforation kann auch in besonders vorteilhafter Weise die im optischen Teil innenliegende Ausgestaltung des Amplitudengitters geschaffen werden. Das Amplitudengitter liegt bei einer diesbezüglich vorteilhaften Ausführung somit vollständig im Inneren des optischen Teils und tritt daher nicht als Oberflächenstruktur auf. Durch diese Ausgestaltung ist das Amplitudengitter auch für sich betrachtet vollständig von dem Werkstoff des optischen Teils umgeben und durch Polymerisation des Werkstoffs des optischen Teils durch Einwirken der Laserstrahlung erst gebildet. Diese innenliegende und somit eingebettete Ausgestaltung des Amplitudengitters ermöglicht die oben genannten Vorteile im besonderen Maße und schafft darüber hinaus auch noch die Situation, dass das Amplitudengitter vor unerwünschten mechanischen Einwirkungen geschützt ist. Gerade beim Herstellen der künstlichen Augenlinse und bei dann erfolgter weiterer Lagerung vor dem Implantieren der künstlichen Augenlinse können daher keine direkten mechanischen Einwirkungen auf dieses Amplitudengitter erfolgen und somit auch keine Beschädigungen dieses Amplitudengitters hervorgerufen werden.

In einer weiteren vorteilhaften Ausführung weist das Amplitudengitter einen ersten Gitterbereich auf, der mit einer ersten Perforationsdichte von Perforationszonen der Mikroperforation und/oder mit einer ersten Dimensionierung von Perforationszonen der Mikroperforation ausgebildet ist. Das Amplitudengitter weist insbesondere einen zweiten Gitterbereich auf, der mit einer zur ersten Perforationsdichte von Perforationszonen unterschiedlichen zweiten Perforationsdichte von Perforationszonen der Mikroperforation und/oder mit einer zur ersten Dimensionierung von Perforationszonen der Mikroperforation unterschiedlichen zweiten Dimensionierung von Perforationszonen der Mikroperforation ausgebildet ist. Damit lassen sich hochpräzise und somit optisch hochfunktionelle Amplitudengitterbereiche schaffen, die auch relativ klein und/oder formspezifisch individuell ausgebildet werden können. Damit kann das Amplitudengitter auch mit unterschiedlichen Abschwächungsgraden beziehungsweise Grauwerten und somit mit ganz individuellen Absorptionswerten für das einfallende Licht ausgebildet werden. Die oben genannten unerwünschten optischen Beugungs- und Reflexionseffekte können dadurch weiter reduziert werden.

Insbesondere ist es auch möglich, eine Lage und/oder eine Anzahl von lichtundurchlässigen Gitterbereichen des Amplitudengitters abhängig von diesen Parametern, nämlich der Perforationsdichte der Perforationszone der Mikroperforation und/oder der Dimensionierung von Perforationszonen der Mikroperforation auszubilden. Die Dimensionierung einer Perforationszone kann ein Innenmaß und/oder eine Tiefe sein.

Je nach Formgebung einer derartigen Perforationszone, die blasenförmig sein kann, können somit individuelle Ausgestaltungen des Amplitudengitters realisiert sein.

In einer vorteilhaften Ausführung kann vorgesehen sein, dass zumindest eine Perforationszone der Mikroperforation zumindest bereichsweise mit einem Farbstoff gefüllt ist. Durch eine derartige zusätzliche Werkstoffzugabe kann das Amplitudengitter nochmals verbessert und mit unterschiedlichen und feiner abgestuften Abschwächungsgraden beziehungsweise Grauwerten realisiert werden. Die Variabilität und Flexibilität der Gitterstruktur ist darüber hinaus erhöht. Nicht zuletzt ist auch eine besonders hohe Präzision der Gitterstrukturen im Mikrometerbereich einschreibbar. Durch spezifische absorbierende Farbstoffe kann hier die optische Funktionalität des Amplitudengitters ganz individuell beeinflusst werden. Es können somit einzelne Perforationszonen mit diesem Farbstoff und/oder unterschiedlichen Farbstoffen, die unterschiedlich absorbieren, zumindest bereichsweise gefüllt sein. Die primäre Funktionalität eines Amplitudengitters, nämlich individuelles Absorptionsverhalten in unterschiedlichen Bereichen, lässt sich hier in hochvariabler und äußerst flexibler Weise individuell gestalten. Dies ist insbesondere auch durch die Gestaltung des Amplitudengitters als Laserstruktur ermöglicht, da hier auch unterschiedliche Perforationszonendichten und unterschiedliche Größen der Perforationszonen sehr genau und somit lagepräzise und formpräzise gestaltet werden können. Die dann gegebenenfalls individuelle Befüllung mit einem oder mehreren Farbstoffen, die individuelles Absorptionsverhalten aufweisen, ermöglicht auch, das besonders unterschiedliche und fein dosierte Absorptionsverhalten eines Amplitudengitters ausgebildet werden können.

In einer vorteilhaften Ausführung ist der Farbstoff in zumindest einer Perforationszone polymerisiert. Dadurch wird die Langzeitstabilität des Farbstoffs verbessert. Eine Polymerisation kann beispielsweise durch UV (Ultraviolett)-Licht oder durch eine Multiphotonenpolymerisation mit Laserlicht erfolgen.

Insbesondere ist eine Lage und/oder eine Anzahl von lichtundurchlässigen Gitterbereichen des Amplitudengitters abhängig von der Art des Farbstoffs und/oder abhängig von der Menge des Farbstoffs und/oder abhängig von der Anzahl der mit Farbstoff zumindest bereichsweise gefüllten Perforationszonen und/oder abhängig von der Lage der mit Farbstoff zumindest bereichsweise gefüllten Perforationszonen ausgebildet. Die bereits oben dargelegte hohe Variabilität und der Individualisierungsgrad eines erzeugbaren Amplitudengitters sind dadurch wesentlich erhöht. Dies ist insbesondere durch die Ausgestaltung des Amplitudengitters als Laserstruktur ermöglicht, da mit einem Laser die bereits oben umfänglich dargelegten Vorteile bezüglich der grundsätzlichen Erzeugung eines derartigen Amplitudengitters als auch bezüglich deren Lagegenauigkeit und hoher optischer Funktionalität erst erreicht ist.

In einer vorteilhaften Ausführung weist das Amplitudengitter um eine optische Hauptachse des optischen Teils zumindest bereichsweise umlaufende Gitterringe als Gitterbereiche auf. Durch eine derartige Strukturierung des Amplitudengitters können spezifisch absorbierend wirkende Ringzonen geschaffen werden, die dann auch eine vorzugsweise geometrisch betrachtet symmetrische Ausgestaltung um die optische Hauptachse aufweisen und in dieser azimutalen Richtung eine einheitliche Wirkung bezüglich der optischen Eigenschaft aufweisen.

Insbesondere ist das Amplitudengitter vollständig innenliegend in dem optischen Teil ausgebildet. Dies bedeutet, dass es vollständig von dem Werkstoff des optischen Teils umgeben ist und somit an den optischen Seiten des optischen Teils nicht außenliegend und somit freiliegend ausgebildet ist. Die dadurch erreichbaren Vorteile sind bereits oben erwähnt.

In einer vorteilhaften Ausführung ist auf zumindest einer Seite des optischen Teils eine zum Amplitudengitter separate optische Gitterstruktur ausgebildet. Durch zwei separate optische Gitterstrukturen können die optischen Abbildungseigenschaften verbessert werden und insbesondere störende optische Effekte nochmals besser unterdrückt werden. Insbesondere können hier auch dann unterschiedliche optische Störeffekte besser unterdrückt werden.

Insbesondere ist die weitere separate optische Gitterstruktur ein Phasengitter. Phasengitter sind optische Beugungsgitter, welche die Phase der einlaufenden Lichtwelle beeinflussen. Im Unterschied zu einem Amplitudengitter, welches ein absorbierendes optisches Gitter ist, ist ein Phasengitter ein Wellenumformgitter, welches die Wellenfront der eintreffenden Lichtquelle umformt. Ein Phasengitter kann auch als Transmissionsgitter oder als Reflexionsgitter ausgebildet sein. Gerade in Kombination mit einem Amplitudengitter können die optischen Abbildungseigenschaften verbessert werden und die eingangs genannten störenden Beugungs- oder Reflexionseffekte verbessert reduziert werden, insbesondere auch zu den genannten Halos und Glares, die im Wesentlichen durch das Amplitudengitter zumindest deutlich reduziert werden können, unterschiedliche störende Beugungseffekte und Reflexionseffekte reduziert werden.

In einer weiteren vorteilhaften Ausführung ist vorgesehen, dass dieses Phasengitter für zumindest zwei Wellenlängen achromatisiert ist. Dadurch ist die Beugungseffizienz für bestimmte Beugungsordnungen maximiert. Dazu wird möglichst viel Intensität in spezifische gewünschte Beugungsordnungen konzentriert, in den übrigen Beugungsordnungen, beispielsweise auch in der nullten Beugungsordnung, hingegen minimiert. Durch vorteilhafte Ausführung ist nun erreicht, dass in spezifischen Gitterbereichen dieses Phasengitters eine spezifische Brechzahlmodifikation ausgebildet ist, um diese Achromatisierung für zumindest zwei Wellenlängen auszubilden. Diese Brechzahlmodifikation ist mit einem Laser, insbesondere einem Ultrakurzpulslaser, erzeugt. Durch eine Achromatisierung wird die Phasendifferenz der interferierenden Wellen von der Wellenlänge unabhängig gemacht. Dies erfolgt bei der genannten vorteilhaften Ausführung für zumindest zwei unterschiedliche Wellenlängen.

Das oben genannte Phasengitter kann beispielsweise ein Blazegitter sein.

Es kann vorgesehen sein, dass zumindest eine optische Seite des optischen Teils, wie sie oben genannt wurden, sphärisch oder asphärisch ausgebildet ist. Es kann eine torische Ausgestaltung und somit ein torisches Oberflächenprofil zumindest auf einer dieser optischen Seite ausgebildet sein. Auch anderweitige optische Oberflächenprofile können auf einer derartigen optischen Seite des optischen Teils ausgebildet sein. Beispielsweise können hier ringförmige Zonen ausgebildet sein, die Bestandteil eines weiteren diffraktiven Elements sein können. Diese ringförmigen Zonen sind dann jedoch auf dieser optischen Seite ausgebildet und somit außenliegende und somit freiliegende optische Strukturelemente.

Insbesondere mit einem Ultrakurzpulslaser einer Laser-Vorrichtung wird der optische Teil der künstlichen Augenlinse derart bearbeitet, dass das Amplitudengitter und gegebenenfalls zumindest ein weiteres optisches Gitter erzeugt werden. Insbesondere werden die Parameter der Laser-Vorrichtung so eingestellt, dass im Fokuspunkt des Laserstrahls eine Laserdisruption im transparenten Kunststoff des optischen Teils der künstlichen Augenlinse kurz über dem Schwellwert auftritt. Wird eine Steigerung der Intensität des Laserstrahls eingestellt, werden größere Schädigungsvolumina in diesem Kunststoff erzeugt. Die meist als Blasenbildung in dem Kunststoff auftretenden Laserdisruptionen können bei ausreichender Energie, beispielsweise im Mikrojoule-Bereich, im Einzelschuss erfolgen oder mit niedriger Energiedichte im Nanojoule-Bereich durch eine hohe Repetitionsrate im Kilohertz- bis Megahertz-Bereich erzeugt werden.

In einer vorteilhaften Ausführung kann auch vorgesehen sein, dass die künstliche Augenlinse eine weitere optische Gitterstruktur aufweist, welche als holographisches Gitter ausgebildet ist. Es kann vorgesehen sein, dass bei einer vorteilhaften Ausführung diese Gitterstruktur ein erstes holographisches Gitter und ein zweites holographisches Gitter aufweist. Insbesondere kann durch diese zwei holographischen Gitter eine Moiré-Struktur erzeugt werden. Vorzugsweise ist ein in Richtung der optischen Hauptachse bemessener Abstand zwischen den beiden holographischen Gittern kleiner dem in Richtung der optischen Hauptachse bemessenen Abstand zwischen der ersten optischen Seite und der zweiten optischen Seite ausgebildet. Insbesondere ist zumindest ein holographisches Gitter im optischen Teil der künstlichen Augenlinse völlig innenliegend zwischen den optischen Seiten des optischen Teils ausgebildet.

Es kann vorgesehen sein, dass auf die erste optische Seite oder auf die zweite optische Seite des optischen Teils zumindest eine Beschichtung aufgebracht ist und zumindest ein holographisches Gitter in dieser Beschichtung ausgebildet ist. Der optische Teil kann zumindest einen laminierten Bereich aufweisen und zumindest ein holographisches Gitter ist in diesem laminierten Bereich ausgebildet.

Insbesondere ist auch dieses holographische Gitter als Laserstruktur in dem optischen Teil ausgebildet.

Durch eine derartige spezifische Ausgestaltung einer Gitterstruktur, insbesondere mit zumindest zwei unterschiedlichen separaten holographischen Gittern, die insbesondere spezifisch überlagert sind, kann eine individuelle Brechkraft der Augenlinse erzeugt werden. Um diese optisch aktive Gitterstruktur, vorzugsweise Moiré-Struktur, innerhalb der Kohärenzlänge des Lichts erzeugen zu können, werden zumindest zwei derartige beugende Strukturen in Form der holographischen Gitter mittels eines Lasers als Laserstruktur erzeugt, insbesondere innerhalb entsprechend benachbarter Schichten eines optischen Teils der Augenlinse. Es kann dabei eine positive als auch eine negative Änderung des Brechungsindex in dem Kunststoff erzeugt werden.

Die künstliche Augenlinse ist insbesondere eine multifokale, insbesondere zumindest trifokale, künstliche Augenlinse. Die künstliche Augenlinse ist insbesondere eine Intraokularlinse.

Des Weiteren betrifft die Erfindung auch ein Verfahren zum Herstellen einer multifokalen, künstlichen Augenlinse gemäß den oben genannten Aspekten, bei welchem eine optisch wirksame Struktur mit einer Laser-Vorrichtung erzeugt wird und ein gepulster Laserstrahl mit einer Pulslänge zwischen 100 fs und 20 ps, einer Wellenlänge zwischen 320 mm und 1.100 mm, eine Pulswiederholrate zwischen 1 kHz und 10 MHz, einem Fokusdurchmesser kleiner 5 µm, insbesondere kleiner 2 µm, und einer Leistungsdichte von größer 10⁶ W/cm², erzeugt wird und auf den Werkstoff der künstlichen Augenlinse einwirkt. Vorzugsweise beträgt die Pulslänge 300 fs und die Wellenlänge vorzugsweise 1.060 nm oder 532 nm oder 355 nm. Durch eine disruptive Bearbeitung kann vorzugsweise eine Wellenlänge von 256 nm und bevorzugt von 213 nm für insbesondere ablative Bearbeitung vorgesehen sein.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder abweichen.

Die in den Unterlagen angegebenen konkreten Werte von Parametern und Angaben zu Verhältnissen von Parametern bzw. Parameterwerten zur Definition von Ausführungsbeispielen der Augenlinse sind auch im Rahmen von Abweichungen, beispielsweise aufgrund von Messfehlern, Systemfehlern, DIN-Toleranzen etc. als vom Rahmen der Erfindung mit umfasst anzusehen, wodurch auch Erläuterungen, die sich auf im Wesentlichen entsprechende Werte und Angaben beziehen darunter zu verstehen sind.

### Kurze Beschreibung der Zeichnungen

Ausführungsbeispiele der Erfindung werden nachfolgend anhand schematischer Zeichnungen näher erläutert. Es zeigen:
- Fig. 1a: eine perspektivische schematische und vereinfachte Darstellung eines ersten Ausführungsbeispiels einer erfindungsgemäßen künstlichen Augenlinse;
- Fig. 1b: eine perspektivische schematische und vereinfachte Darstellung eines weiteren Ausführungsbeispiels einer erfindungsgemäßen künstlichen Augenlinse;
- Fig. 2: eine Draufsicht auf ein Ausführungsbeispiel eines optischen Teils einer künstlichen Augenlinse mit einer spezifischen doppelbrechenden Struktur;
- Fig. 3: eine Draufsicht auf ein Ausführungsbeispiel eines optischen Teils einer künstlichen Augenlinse mit einem zusätzlichen spezifischen ersten Amplitudengitter;
- Fig. 4: eine Draufsicht auf ein Ausführungsbeispiel eines optischen Teils einer künstlichen Augenlinse mit einem zusätzlichen spezifischen zweiten Amplitudengitter;
- Fig. 5: eine schematische Schnittdarstellung eines Ausführungsbeispiels einer künstlichen Augenlinse mit einem zusätzlichen Phasengitter im optischen Teil; und
- Fig. 6: eine vereinfachte Darstellung einer Laser-Vorrichtung zum Herstellen einer künstlichen Augenlinse.

### Bevorzugte Ausführungen der Erfindung

In den Figuren werden gleiche oder funktionsgleiche Elemente mit den gleichen Bezugszeichen versehen.

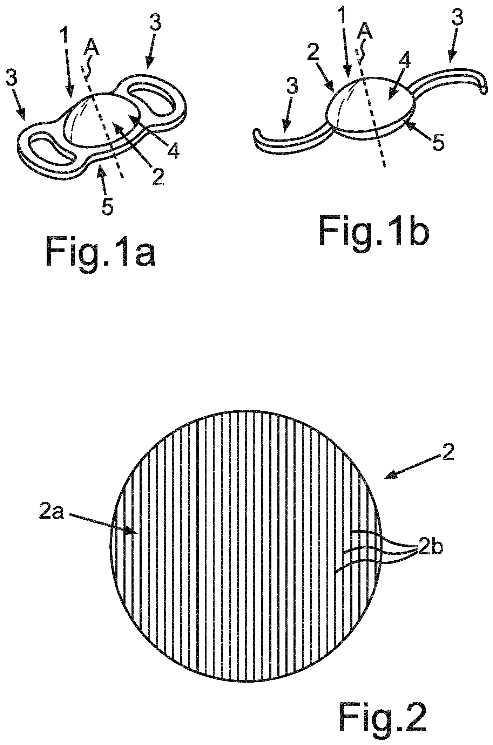

In Fig. 1a ist in einer perspektivischen Darstellung ein erstes Ausführungsbeispiel einer künstlichen Augenlinse 1 gezeigt, die hier eine Intraokularlinse ist. Die nachfolgend als Augenlinse 1 bezeichnete künstliche Augenlinse 1 weist einen optischen Teil 2 und daran anschließend eine Haptik 3 auf. Die Augenlinse 1 ist multifokal, insbesondere trifokal. Die Augenlinse 1 ist faltbar und über einen Kleinschnitt in ein Auge einführbar. Der optische Teil 2, der wesentlich für die optische Abbildungseigenschaft der Augenlinse 1 ist, umfasst eine optische Hauptachse A. Der optische Teil 2 weist darüber hinaus in Richtung dieser optischen Hauptachse A betrachtet eine erste optische Fläche bzw. optische Seite 4 auf, die eine Vorderseite sein kann, und weist gegenüberliegend eine zweite optische Fläche bzw. optische Seite 5 auf, die eine Rückseite sein kann. Die Vorderseite ist im implantierten Zustand der Augenlinse 1 im Auge der Hornhaut zugewandt, wohingegen die Rückseite dieser Hornhaut abgewandt ist.

In Fig. 1b ist in einer perspektivischen Darstellung ein weiteres Ausführungsbeispiel einer als Intraokularlinse ausgebildeten künstlichen Augenlinse 1 gezeigt. Sie unterscheidet sich von der Ausführung in Fig. 1a durch die unterschiedliche Haptik 3. Mittels der Haptik 3 ist die Augenlinse 1 im Auge gehalten.

Die optischen Seiten 4 und 5 sind in den Ausführungen uneben, insbesondere konvex, gekrümmt. Auf zumindest einer optischen Seite 4, 5 ist auf dieser konvexen Grundform ein diffraktives Profil ausgebildet.

Grundsätzlich können auch anderweitig geformte und ausgestaltete Haptiken 3 vorgesehen sein.

In Fig. 2 ist eine vereinfachte und schematische Draufsicht auf den einstückigen optischen Teil 2 einer künstlichen Augenlinse 1 gezeigt. Der optische Teil 2 ist somit als ein einziges Teil hergestellt, und insbesondere somit nicht aus mehreren Einzelteilen zerstörungsfrei unlösbar zusammengesetzt. Insbesondere im Inneren des optischen Teils 2 und somit vollständig in dem Material des optischen Teils 2 eingebettet bzw. von diesem Material umgeben ist eine doppelbrechende Struktur 2a ausgebildet. Die doppelbrechende Struktur 2a ist als Laserstruktur ausgebildet. Insbesondere die Ausgestaltungsart ermöglicht es überhaupt erst, im Inneren des optischen Teils 2 eine solche Struktur zu erzeugen, insbesondere auch dann erst im Nachgang zur erfolgten einstückigen Formherstellung des Grundkörpers des optischen Teils 2. Auch ermöglicht die Ausgestaltung als Laserstruktur grundsätzlich erst eine spezifische Formpräzision der gesamten doppelbrechenden Struktur 2a und Strukturelemente, hier die Linien 2b, in hoher Präzision und mit individuellen, auch minimalen Abständen, als auch im besonderen Maße mit gleichbleibender Geradlinigkeit und Parallelität. Die optische Funktionalität der doppelbrechenden Struktur 2a ist gerade durch die Ausgestaltung als Laserstruktur besonders hoch. Die doppelbrechende Struktur 2a ist insbesondere eine lineare doppelbrechende Struktur. Die doppelbrechende Struktur 2a weist insbesondere eine Mehrzahl von Linien 2b auf, die parallel und beabstandet zueinander ausgebildet sind.

Die doppelbrechenden Struktur 2a ist bei der in Fig. 2 dargestellten Ausführungsform auf der gesamten Oberfläche des optischen Teils 2 ausgebildet. Sie kann auch nur in einem dazu reduzierten Flächenbereich ausgebildet sein. Der Flächenbereich kann dann vielfältige Flächenformen aufweisen und somit auch eine beliebige Freiformfläche aufweisen.

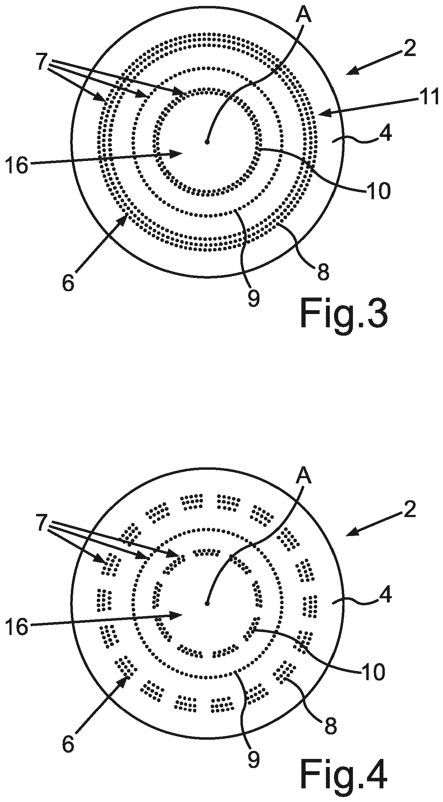

In Fig. 3 ist in einer vereinfachten Darstellung ein weiteres Ausführungsbeispiel gezeigt, bei welchem der optische Teil 2 mit Blick auf die optische Seite 4 gezeigt. Die Augenlinse 1 könnte zusätzlich oder anstatt dazu auch mit Blick auf die optische Seite 5 entsprechend ausgebildet sein. Im Ausführungsbeispiel ist insbesondere zusätzlich zur doppelbrechenden Struktur 2a, vorzugsweise im Inneren des optischen Teils 2, der vorzugsweise diskusförmig ausgebildet ist, zumindest ein diffraktives Gitter beziehungsweise zumindest eine diffraktive Gitterstruktur ausgebildet, die hier insbesondere ein Amplitudengitter 6 ist. Das Amplitudengitter 6 ist als Laserstruktur ausgebildet. Das Amplitudengitter 6 ist insbesondere vollständig innerhalb des optischen Teils 2 angeordnet und durch einen Laser erzeugt. Das Amplitudengitter 6 ist daher vollständig von dem restlichen Werkstoff des optischen Teils 2 umgeben und somit vollständig von diesem Werkstoff umschlossen.

Der optische Teil 2 ist einstückig und somit aus einem einzigen Teil ausgebildet.

Insbesondere kann das Amplitudengitter 6 auch als Mikroperforation 7 in dem optischen Teil 2 ausgebildet sein.

Das Amplitudengitter 6 weist einen ersten Gitterbereich 8 auf, der mit einer ersten Perforationsdichte von Perforationszonen der Mikroperforation 7 und/oder mit einer ersten Dimensionierung von Perforationszonen dieser Mikroperforation 7 ausgebildet ist. Das Amplitudengitter 6 weist vorzugsweise einen dazu separaten zweiten Gitterbereich 9 auf, der mit einer zur ersten Perforationsdichte von Perforationszonen der Mikroperforation 7 unterschiedlichen, zweiten Perforationsdichte von Perforationszonen der Mikroperforation 7 und/oder mit einer zur ersten Dimensionierung von Perforationszonen der Mikroperforation 7 unterschiedlichen zweiten Dimensionierung von Perforationszonen der Mikroperforation 7 ausgebildet ist. Insbesondere ist vorgesehen, dass das Amplitudengitter 6 auch noch zumindest einen dritten Gitterbereich 10 aufweisen kann, der mit einer zur ersten und zweiten Perforationsdichte von Perforationszonen der Mikroperforation 7 und/oder mit einer zur ersten und zweiten Dimensionierung von Perforationszonen der Mikroperforation 7 unterschiedlichen dritten Perforationsdichte von Perforationszonen und/oder Dimensionierung von Perforationszonen der Mikroperforation 7 ausgebildet ist.

Es kann vorgesehen sein, dass sich zumindest ein Gitterbereich 8, 9, 10 in radialer Richtung zur optischen Hauptachse A, die senkrecht auf der Figurenebene steht, wiederholt. Auch eine alternierende Anordnung in dieser radialen Richtung zur optischen Hauptachse A von zumindest zwei Gitterbereichen 8, 9, 10 kann vorgesehen sein.

In der gezeigten Ausführung gemäß Fig. 3 sind die einzelnen Perforationszonen der Mikroperforation 7 als Ringzonen ausgebildet, die insbesondere vollständig umlaufend um die optische Hauptachse A ausgebildet sind. Es kann auch eine Ausgestaltung vorgesehen sein, bei welcher zumindest ein Gitterbereich 8, 9, 10 nur teilweise umlaufend um die optische Hauptachse A ausgebildet ist.

Wie zu erkennen ist, ist auch die radiale Dicke eines Gitterbereichs 8, 9, 10 unterschiedlich ausgebildet.

Insbesondere ist vorgesehen, dass in zumindest einer Perforationszone, vorzugsweise in mehreren Perforationszonen, zumindest eines Gitterbereichs 8, 9, 10 zumindest ein Farbstoff enthalten ist. Das Absorptionsverhalten des Amplitudengitters 6 kann somit unterschiedlich eingestellt werden.

Es kann vorgesehen sein, dass der zumindest eine absorbierende Farbstoff in zumindest einer Perforationszone polymerisiert ist.

In einer vorteilhaften Ausführung ist vorgesehen, dass auf der optischen Seite 4 und/oder auf der optischen Seite 5 und somit außenliegend zusätzlich eine weitere optische Gitterstruktur als zum Amplitudengitter 6 separate Gitterstruktur ausgebildet ist. Diese weitere Gitterstruktur ist als separates optisches Gitter ausgebildet und insbesondere als Phasengitter 11 ausgebildet. Der Übersichtlichkeit dienend ist dieses Phasengitter 11 in Fig. 2 nicht explizit strukturell gezeigt, sondern lediglich mit einem Bezugszeichen angedeutet.

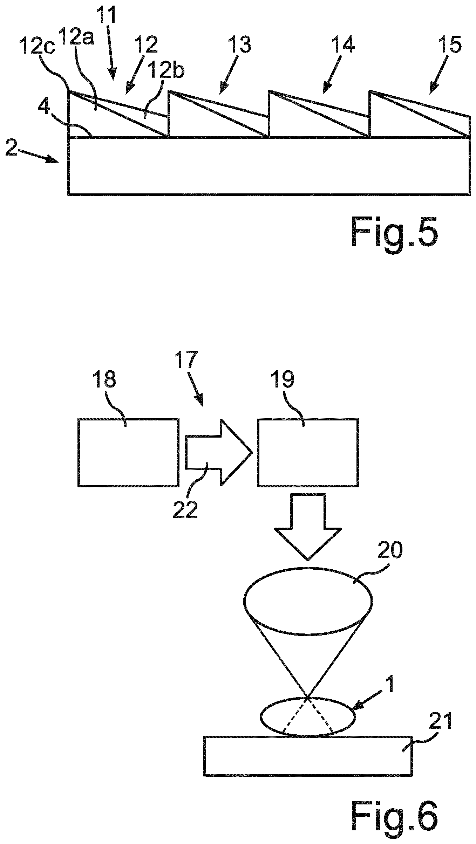

Für eine mögliche Ausführungsform des Phasengitters 11 wird auf die sehr vereinfachte und schematische Darstellung in Fig. 5 verwiesen. Dort ist ein Ausschnitt des optischen Teils 2 gezeigt und es ist eine sehr vereinfachte Schnittdarstellung durch den optischen Teil 2 gezeigt, wobei die optische Hauptachse A in dieser Schnittebene liegt.

Als Beispiel ist hier das Phasengitter 11 als Blazegitter gezeigt. Insbesondere sind die optische Seite 4 und die optische Seite 5 jeweils uneben ausgebildet, insbesondere gekrümmt ausgebildet, wobei hier eine sphärische oder asphärische Krümmung ausgebildet sein kann. Bei der stark vergrößerten Querschnittsansicht in Fig. 5 ist die optische Seite 4 nicht gekrümmt, sondern vereinfacht in planem Zustand dargestellt.

In Fig. 5 ist ein Beispiel gezeigt, in welchem das Phasengitter 11 auf der optischen Seite 4 ausgebildet ist. Auch dieses Phasengitter 11 ist als Laserstruktur ausgebildet und mit der nachfolgend noch erläuterten Laser-Vorrichtung erzeugt. Das Phasengitter 11 weist mehrere Gitterbereiche 12, 13, 14 und 15 auf. Die Gitterbereiche 12 bis 15 sind in Anzahl als auch in ihrer individuellen Formgestaltung lediglich als Beispiel und schematisch und nicht abschließend zu verstehen. Die Gitterbereiche 12 bis 15 sind als gestufte Zonen zueinander ausgebildet. Es ist vorzugsweise vorgesehen, dass das Phasengitter 11 für zumindest zwei Wellenlängen achromatisiert ist.

Insbesondere ist vorgesehen, dass der Gitterbereich 12 einen ersten Teilbereich 12a und einen zweiten Teilbereich 12b aufweist. Die beiden Teilbereiche 12a und 12b weisen unterschiedliche Brechzahlen auf. Dies ist durch Einwirkung mit einem Laserstrahl auf den Werkstoff des optischen Teils 2 erzeugt. Grundsätzlich ist in vorteilhafter Weise das Phasengitter 11 aus dem gleichen Werkstoff wie der optische Teil 2 ausgebildet. Unter Einwirkung mit einem Laserstrahl wird insbesondere der weiter außen liegende Teilbereich 12b derart beeinflusst, dass sich die Brechzahl ändert, wobei hier durch diesen Laserstrahl eine Veränderung der Werkstoffkonfiguration erzeugt wird und sich daraus die Brechzahländerung ergibt. Der Teilbereich 12a hingegen weist insbesondere die unveränderte Brechzahl entsprechend dem Werkstoff des optischen Teils 2 auf. Wie in der ebenfalls nicht einschränkend zu verstehenden Ausführungsform in Fig. 5 zu erkennen ist, ist der äußere und in seiner Brechzahl durch Einwirkung des Laserstrahls veränderte Teilbereich 12b in dieser Querschnittdarstellung dreieckförmig ausgebildet. Er weitetet sich von einer Zonenspitze 12c her betrachtet zu dem benachbarten zweiten Gitterbereich 13 hin auf und er weist insbesondere dann an der vorzugsweise ausgebildeten Anmündung an dem benachbarten Gitterbereich 13 seine größte Aufweitung auf.

Insbesondere ist eine entsprechende Ausgestaltung auch bei zumindest einem weiteren Gitterbereich 13 bis 15 ausgebildet, wie dies ebenfalls in Fig. 5 angedeutet ist.

In Fig. 4 ist eine ebenfalls vereinfachte Darstellung des optischen Teils 2 eines weiteren Ausführungsbeispiels der künstlichen Augenlinse 1 gezeigt. Im Unterschied zur Darstellung gemäß Fig. 3 ist hier vorgesehen, dass die Ausgestaltung des zur doppelbrechenden Struktur 2a zusätzlichen Amplitudengitters 6 nicht mit umlaufenden Ringen als Gitterbereiche 8, 9, 10 ausgebildet ist, sondern dass diese durch mehrere einzelne Lokalbereiche, die in Umlaufrichtung um die optische Hauptachse A beabstandet und vorzugsweise äquidistant zueinander ausgebildet sind, erzeugt ist. Auch hier sind die einzelnen Lokalbereiche durch jeweils mehrere Perforationszonen, die ebenfalls insbesondere mit einem oder mehreren Farbstoffen zumindest bereichsweise gefüllt sein können, gebildet. Wie hier zu erkennen ist, weist der Gitterbereich 8 mehrere Lokalbereiche auf, die in einer individuellen Ausgestaltung unterschiedlich zu Lokalbereichen des beispielsweise weiter innen liegenden Gitterbereichs 10 gestaltet sind. Insbesondere kann auch vorgesehen sein, dass ein Gitterbereich, hier der Gitterbereich 9, entsprechend der Ausgestaltung in Fig. 3 realisiert ist. Ebenso kann eine weitere Alternative dadurch ausgebildet sein, dass beispielsweise der Gitterbereich 8 entsprechend in Fig. 3 ausgebildet ist und nur der Gitterbereich 10 gemäß der Darstellung in Fig. 4 ausgebildet ist. Ebenso kann es vorgesehen sein, dass der Gitterbereich 10 entsprechend der Ausgestaltung in Fig. 3 realisiert ist und nur der Gitterbereich 8 entsprechend der Ausgestaltung in Fig. 4 realisiert ist. Ebenso kann es vorgesehen sein, dass der Gitterbereich 9 entsprechend unterbrochen in einzelnen Lokalbereichen gestaltet ist, wie dies bei den Ausführungen in Fig. 4 für die Gitterbereiche 8 und 10 vorgesehen ist.

Zusätzlich zu dem Amplitudengitter 6 und zusätzlich oder anstatt des gegebenenfalls vorhandenen Phasengitters 11 kann auch noch eine weitere optisch wirksame Gitterstruktur 16 (Fig. 3 und Fig. 4) an oder in dem optischen Teil 2 ausgebildet sein. Diese weitere separate optische Gitterstruktur 16 weist zumindest ein holographisches Gitter auf. Diese weitere optische Gitterstruktur 16 ist vorzugsweise als Laserstruktur ausgebildet und insbesondere mit der Laser-Vorrichtung, wie sie nachfolgend zu Fig. 6 erläutert wird, erzeugt.

Vorzugsweise weist diese optische Struktur 16 zwei separate holographische Gitter, ein erstes holographisches Gitter und ein zweites holographisches Gitter, auf. Vorzugsweise ist ein in Richtung der optischen Hauptachse A bemessener Abstand zwischen den beiden holographischen Gittern kleiner dem in Richtung der optischen Hauptachse A bemessenen Abstand zwischen der ersten optischen Seite 4 und der zweiten optischen Seite 5. In einer vorteilhaften Ausführung sind die zumindest zwei holographischen Gitter überlagert und insbesondere so überlagert, dass eine Moiré-Struktur ausgebildet ist. Insbesondere ist diese optische Struktur 16 als zentrale Kreiszone um die optische Hauptachse A des optischen Teils 2 ausgebildet.

In Fig. 6 ist in einer schematischen Darstellung eine Laser-Vorrichtung 17 gezeigt, welche zum Herstellen einer multifokalen, künstlichen Augenlinse 1 ausgebildet ist. Insbesondere ist mit dieser Laser-Vorrichtung 17 die Erzeugung des Amplitudengitters 6 und/oder des Phasengitters 11 und/oder der zumindest einen weiteren optischen Gitterstruktur 16 ermöglicht. Die Laser-Vorrichtung 17 weist zumindest einen Laser 18 auf, welcher ein Ultrakurzpulslaser ist. Diese Laser-Vorrichtung 17 weist einen insbesondere dreidimensional einstellbaren Scanner 19 auf, mittels welchem ein gepulster Laserstrahl des Lasers 18 einstellbar ist. Des Weiteren weist die Laser-Vorrichtung 17 eine Fokussieroptik 20 auf, die im Strahlgang dem Scanner 19 nachrangig angeordnet ist. Die Laser-Vorrichtung 17 weist darüber hinaus eine Aufnahme 21 auf, auf welcher die künstliche Augenlinse 1 aufgebracht wird, um dann die gewünschte Strukturierung mit dem durch die Fokussieroptik 20 fokussierten Laserstrahl 22 einwirken lassen zu können. Der gepulste Laserstrahl 22 mit seinen Laserpulsen wird insbesondere mit einer Pulslänge zwischen 100 fs und 20 ps, insbesondere einer Wellenlänge zwischen 200 nm und 1.100 nm, insbesondere einer Pulswiederholrate zwischen 1 kHz und 10 MHz, insbesondere einem Fokusdurchmesser von kleiner als 5 µm und insbesondere einer Leistungsdichte von größer 10⁸W/cm² erzeugt. Es wird hier insbesondere eine Multiphotonenabsorption ermöglicht. Die Fokussieroptik 20 kann eine numerischen Apertur von größer als 0,1, vorzugsweise größer als 0,3 und insbesondere größer als 0,5, aufweisen. Mit der Laser-Vorrichtung 17 ist auch das Erzeugen von Fokusdurchmessern von kleiner als 5 µm, insbesondere kleiner als 2 µm, möglich. Dabei ist eine Leistungsdichte des fokussierten Laserstrahls von größer als 10¹⁰ W/cm² sinnvoll, um einen optischen Durchbruch (Photodisruption) des Polymerwerkstoffes der künstlichen Augenlinse zu erreichen, wenn beispielsweise keine lineare Absorption des Polymerwerkstoffes diesen Effekt unterstützt. Um lediglich eine nicht-lineare Wechselwirkung im Polymerwerkstoff der künstlichen Augenlinse 1 zu erzielen, ist auch eine Leistungsdichte von kleiner als 10¹⁰ W/cm² vorgesehen, die nicht zur Photodisruption führt, jedoch optische und/oder mechanische beziehungsweise damit einhergehende auch hygroskopische Werkstoffeigenschaften ändern kann. Um eine hohe Bearbeitungseffizienz der künstlichen Augenlinse sicherzustellen, ist eine Repetitionsrate der ultrakurzen Laserpulse des Laserstrahls 22 im Bereich von 1 kHz bis 10 MHz vorteilhaft. Dabei werden Pulsenergien im sub-µJ-Bereich benutzt. Insbesondere bei einer Repetitionsrate von größer als 1 MHz ist aufgrund kumulativer Wechselwirkungseffekte auch eine Pulsenergien von kleiner als 1 µJ vorgesehen.

## Patentansprüche

1. Künstliche Augenlinse (1) mit einem optischen Teil (2), der eine in Richtung einer optischen Hauptachse (A) der Augenlinse (1) betrachtet erste optische Seite (4) und eine gegenüberliegende zweite optische Seite (5) aufweist, wobei der optische Teil (2) mit einer eine Doppelbrechung aufweisenden Struktur (2a) ausgebildet ist,
**dadurch gekennzeichnet, dass**
die doppelbrechende Struktur (2a) in dem einstückigen optischen Teil (2) als Laserstruktur ausgebildet ist.

2. Künstliche Augenlinse (1) nach Anspruch 1,
**dadurch gekennzeichnet, dass**
der optische Teil (2) aus einem isotropen Material ausgebildet ist.

3. Künstliche Augenlinse (1) nach Anspruch 1 oder 2,
**dadurch gekennzeichnet, dass**
die doppelbrechende Struktur (2a) eine lineare Struktur ist.

4. Künstliche Augenlinse (1) nach Anspruch 3,
**dadurch gekennzeichnet, dass**
die doppelbrechende Struktur (2a) gerade Linien (2b) aufweist.

5. Künstliche Augenlinse (1) nach einem der vorhergehenden Ansprüche,
**dadurch gekennzeichnet, dass**
der optische Teil (2) zumindest eine zur doppelbrechenden Struktur (2a) separate weitere diffraktive Gitterstruktur (6, 11, 16) als Laserstruktur aufweist.

6. Künstliche Augenlinse (1) nach Anspruch 5,
**dadurch gekennzeichnet, dass**
die diffraktive Gitterstruktur (6, 11, 16) ein Amplitudengitter (6) aufweist.

7. Künstliche Augenlinse (1) nach Anspruch 5 oder 6,
**dadurch gekennzeichnet, dass**
die diffraktive Gitterstruktur (6, 11, 16) ein Phasengitter (11) aufweist.

8. Künstliche Augenlinse (1) nach einem der vorhergehenden Ansprüche,
**dadurch gekennzeichnet, dass**
die diffraktive Gitterstruktur (6, 11, 16) zumindest ein holografisches Gitter (16) aufweist.

9. Verfahren zum Herstellen einer künstlichen Augenlinse (1) nach einem der vorhergehenden Ansprüche, bei welchem die doppelbrechende Struktur (2a) mit einer Laser-Vorrichtung (17) erzeugt wird, und ein gepulster Laserstrahl mit einer Pulslänge zwischen 100 fs und 20 ps, einer Wellenlänge zwischen 320 nm und 1100 nm, eine Pulswiederholrate zwischen 1 kHz und 10 Mhz, einem Fokusdurchmesser kleiner 5 µm und einer Leistungsdichte von größer 10⁶ W/cm² erzeugt wird und auf den optischen Teil (2) einwirkt.

## Claims

1. Artificial eye lens (1) having an optical part (2) which has, viewed in the direction of an optical principal axis (A) of the eye lens (1), a first optical side (4) and an opposite, second optical side (5), wherein the optical part (2) is formed with a structure (2a) having birefringence,
**characterized in that**
the birefringent structure (2a) in the integral optical part (2) is formed as a laser structure.

2. Artificial eye lens (1) according to Claim 1, **characterized in that**
the optical part (2) is formed from an isotropic material.

3. Artificial eye lens (1) according to Claim 1 or 2, **characterized in that**
the birefringent structure (2a) is a linear structure.

4. Artificial eye lens (1) according to Claim 3, **characterized in that**
the birefringent structure (2a) has straight lines (2b).

5. Artificial eye lens (1) according to one of the preceding claims,
**characterized in that**
the optical part (2) has at least one further diffractive grating structure (6, 11, 16), which is separate from the birefringent structure (2a), as a laser structure.

6. Artificial eye lens (1) according to Claim 5, **characterized in that**
the diffractive grating structure (6, 11, 16) has an amplitude grating (6).

7. Artificial eye lens (1) according to Claim 5 or 6, **characterized in that**
the diffractive grating structure (6, 11, 16) has a phase grating (11).

8. Artificial eye lens (1) according to one of the preceding claims,
**characterized in that**
the diffractive grating structure (6, 11, 16) has at least one holographic grating (16).

9. Method for producing an artificial eye lens (1) according to one of the preceding claims, in which the birefringent structure (2a) is produced with a laser apparatus (17), and a pulsed laser beam having a pulse length of between 100 fs and 20 ps, a wavelength of between 320 nm and 1100 nm, a pulse repetition rate of between 1 kHz and 10 MHz, a focus diameter of less than 5 µm, and a power density of greater than 10⁶ W/cm² is produced and acts on the optical part (2).

## Revendications

1. Lentille oculaire artificielle (1) comportant une partie optique (2), qui comporte un premier côté optique (4) vu dans la direction d'un axe optique principal (A) de la lentille oculaire (1) et un deuxième côté optique opposé (5), la partie optique (2) étant conçue avec une structure (2a) présentant une biréfringence, **caractérisée en ce que**
la structure biréfringente (2a) dans la partie optique monobloc (2) est réalisée sous la forme d'une structure laser.

2. Lentille oculaire artificielle (1) selon la revendication 1, **caractérisée en ce que**
la partie optique (2) est une matière isotrope.

3. Lentille oculaire artificielle (1) selon la revendication 1 ou 2,
**caractérisée en ce que**
la structure biréfringente (2a) est une structure linéaire.

4. Lentille oculaire artificielle (1) selon la revendication 3,
**caractérisée en ce que**
la structure biréfringente (2a) comporte des lignes droites (2b).

5. Lentille oculaire artificielle (1) selon l'une des revendications précédentes,
**caractérisée en ce que**
la partie optique (2) comporte au moins une structure de réseau de diffraction (6, 11, 16) supplémentaire, séparée de la structure biréfringente (2a), comme structure laser.

6. Lentille oculaire artificielle (1) selon la revendication 5,
**caractérisée en ce que**
la structure de réseau de diffraction (6, 11, 16) comporte un réseau d'amplitude (6).

7. Lentille oculaire artificielle (1) selon la revendication 5 ou 6,
**caractérisée en ce que**
la structure de réseau de diffraction (6, 11, 16) comporte un réseau de phase (11).

8. Lentille oculaire artificielle (1) selon l'une des revendications précédentes,
**caractérisée en ce que**
la structure de réseau de diffraction (6, 11, 16) comporte au moins un réseau holographique (16).

9. Procédé de fabrication d'une lentille oculaire artificielle (1) selon l'une des revendications précédentes, dans lequel la structure biréfringente (2a) est générée avec un dispositif laser (17), et un faisceau laser pulsé est généré avec une longueur d'impulsion comprise entre 100 fs et 20 ps, une longueur d'onde comprise entre 320 nm et 1 100 nm, un taux de répétition d'impulsions compris entre 1 kHz et 10 MHz, un diamètre de foyer inférieur à 5 um et une densité de puissance supérieure à 10⁶ W/cm² et agit sur la partie optique (2).
